# EUROPEAN PATENT SPECIFICATION

(11) **EP 3 190 993 B1**
(45) Date of publication and mention of the grant of the patent: **12.04.2023**
(21) Application number: 15770678.9
(22) Date of filing: 31.08.2015
(51) Int. Cl.: A61B 17/32

(54) **TUMOR MARGIN DEVICE**
TUMORRANDVORRICHTUNG
DISPOSITIF DE MARGE TUMORALE

(30) Priority: 08.09.2014 US 201414479720
(43) Date of publication of application: 19.07.2017
(73) Proprietor: Medtronic Xomed, Inc., Jacksonville, FL 32216 (US)
(72) Inventor: NGUYEN, Thoai, Jacksonville, Florida 32210 (US); BERMAN, Phillip J., Jacksonville, Florida 32257 (US)
(74) Representative: Dehns
(86) International application number: PCT/US2015/047724
(87) International publication number: WO 2016/040033

(56) References cited:
- WO-A1-2008/124650
- US-A- 4 274 414
- US-A- 4 274 414
- US-A- 5 376 078
- US-A1- 2009 124 975
- US-A1- 2009 124 975
- US-A1- 2010 198 242
- US-A1- 2010 198 242
- US-A1- 2012 065 658
- US-A1- 2012 065 658
- US-A1- 2012 191 117
- US-B1- 6 217 598
- US-B1- 6 217 598

## Description

### Background

The present disclosure relates to treatment of tumors on or near the brain or spine. More particularly, it relates to surgical systems, instruments, and exemplary methods useful in reducing and/or removing tumors and fibrous tissues.

Neurosurgery is the treatment of choice for accessible brain tumors. The goal of surgery is to remove as much tumor tissue as possible. The most commonly performed surgery for removal of a brain tumor is a craniotomy. In general, the neurosurgeon makes an incision into the scalp, cranium, dura, meninges, and cortex to expose an area of brain over the tumor. Location and removal of the tumor then takes place. In this regard, a variety of surgical instruments, such as a cavitational ultrasonic surgical aspirator (CUSA) or a surgical laser knife, are commonly used.

The delicate tissues associated with the human brain anatomy give rise to several concerns when using a CUSA, laser knife, or other brain surgery instrument. By way of reference, the brain is covered by three membranes or meninges that in turn are surrounded by the skull. The three layers of meninges are the dura mater (immediately beneath the skull), the arachnoid, and the pia mater. Spinal fluid flows in the space between the arachnoid and the pia mater membranes, known as the subarachnoid space. These meninges are thin and delicate, with the pia mater carrying or maintaining the many blood vessels associated with the brain. Due to the friable nature of especially the pia mater, neurosurgeons must exercise great care when attempting to surgically remove a brain tumor; unintended damage to the pia mater can diminish the blood supply to the brain. Unnecessary injury to other healthy structures, such as the arachnoid or brain tissue (e.g., cerebral cortex) can also lead to patient impairment. With this in mind, CUSA instruments deliver ultrasonic action to remove tissue and bone. The surgeon attempts to place the ultrasonic cutting tip against tissue to be destroyed. However, high frequency cutting may also occur and damage tissue surrounding the targeted tumor when touched by the instrument's shaft. Further, due to the relatively large size of the CUSA handpiece, it may be difficult to visually confirm placement of the ultrasonic shaft/tip. Similarly, use of a laser knife may give rise to unintended tissue damage with thermal insult or thermal collateral of surrounding viable tissue due to local heat in and around the incision line. Additionally, ultrasonic devices can cause a spray of cancerous matter during treatment.

Further, typical oscillating microdebrider blades cause jerking of the blade as the blade rotation changes direction. These microdebrider blades often tear off chunks of tissue and do not provide clean, straight cuts.

US2012/065658 A1, US2010/198242 A1 and US6217598 B1 are examples of known surgical cutting instruments in the field.

In light of the above, a need exists for surgical systems and methods for reducing or removing brain tumors while minimizing the likelihood of normal tissue damage.

### Summary

A cutting device for use with a powered surgical tool is defined in claim 1.

A cutting device for use with a powered surgical tool is defined in claim 9.

Preferred embodiments are defined in the dependent claims.

### Brief Description of the Drawings

FIG. 1 is a perspective view of a surgical cutting instrument for surgically reducing or removing a tumor or fibrous tissue in accordance with principles of the present disclosure;
FIG. 2 is an exploded view of the cutting implement of FIG. 1;
FIGS. 3A and 3B are partial side and cross-sectional views of the cutting implement of FIG. 1;
FIGS. 4A and 4B are enlarged cross-sectional and perspective views of an inner hub assembly of the assembly of FIG. 2;
FIGS. 5A and 5B are enlarged side and cross-sectional views of an outer hub assembly of the assembly of FIG. 2;
FIGS. 6A through 6C are enlarged side, top, and end views of a distal portion of an inner blade of the cutting implement according to the invention;
FIGS. 7A through 7C are enlarged side, top, and end views of a distal region of an outer blade of a cutting implement according to one embodiment of the present disclosure;
FIGS. 8A and 8B are enlarged cross-sectional and perspective views of an assembled distal portion of the inner blade of FIGS. 6A through 6C and distal region of the outer blade of FIGS. 7A through 7C of a cutting implement in accordance with principles of the present disclosure;
FIGS. 9A and 9B are enlarged perspective and top views of a distal portion of an inner blade of the cutting implement according to the invention;
FIGS. 10A and 10B are enlarged side and cross-sectional views of a distal region of an outer blade of a cutting implement according to another embodiment of the present disclosure;
FIGS. 11A and 11B are enlarged perspective and cross-sectional views of an assembled distal portion of the inner blade of FIGS. 9A and 9B and the distal region of the outer blade of FIGS. 10A and 10B of a cutting implement in accordance with principles of the present disclosure.

### Detailed Description

Surgical instruments embodying principles of the present disclosure can be employed in various types of surgery including, but not limited to, neurosurgery or spinal surgery on the dura or spinal column.

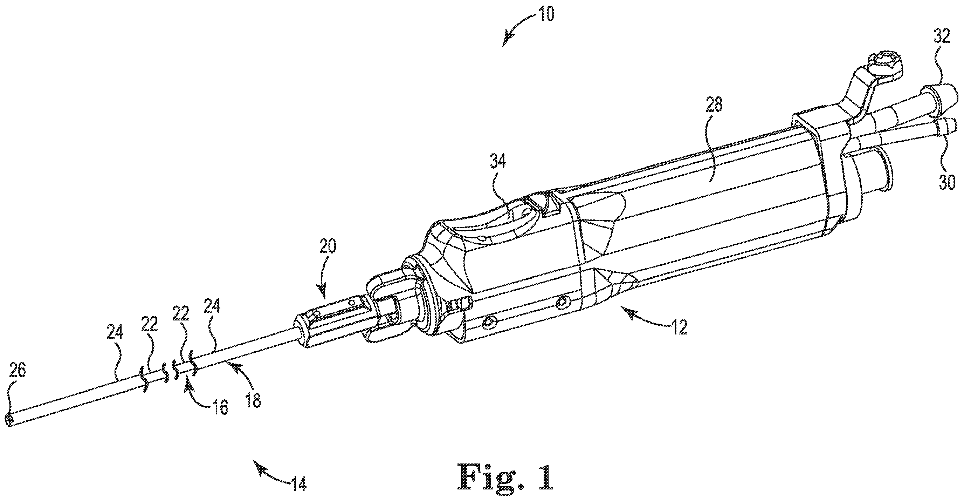

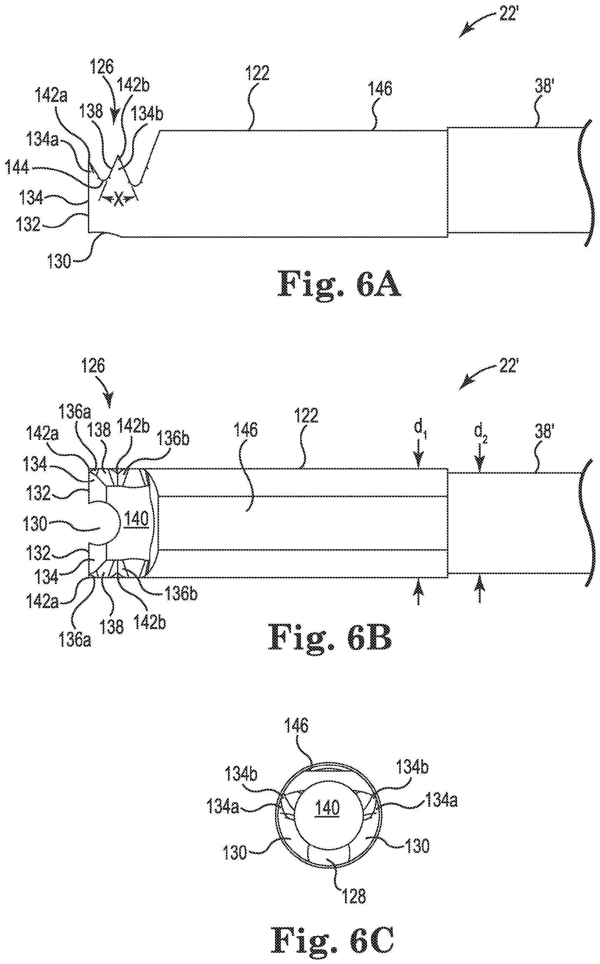

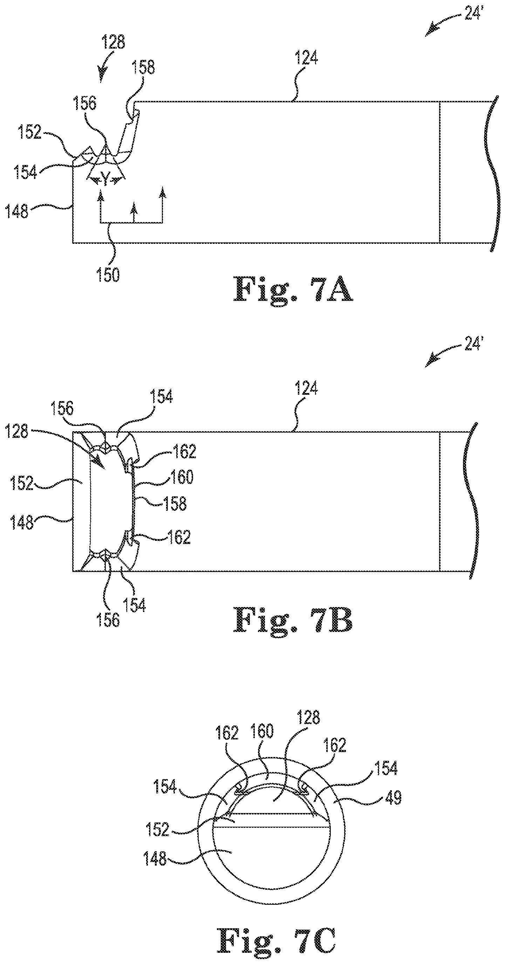

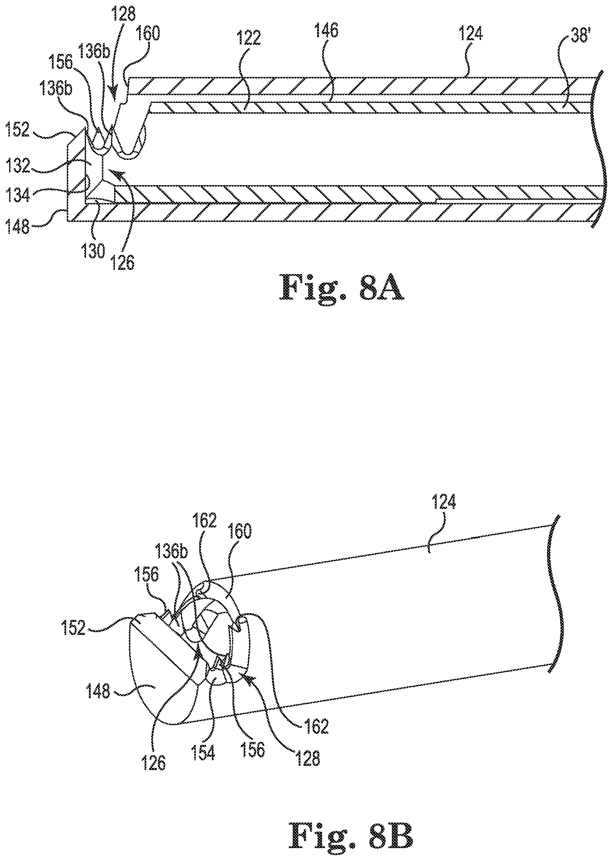

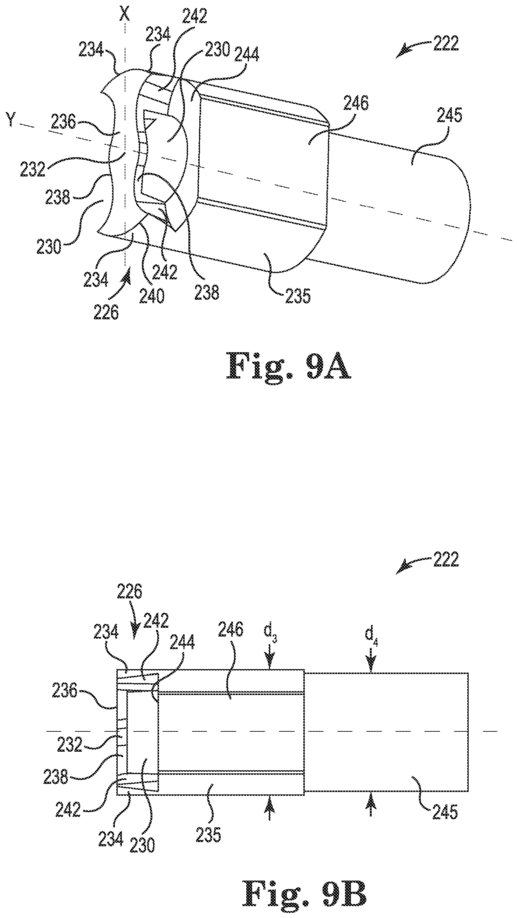

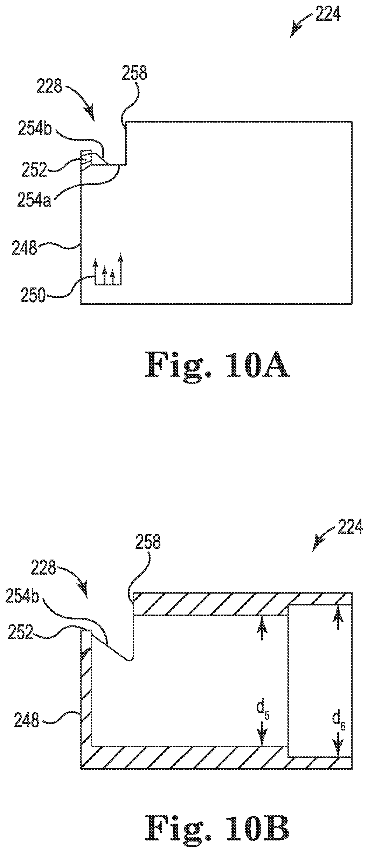

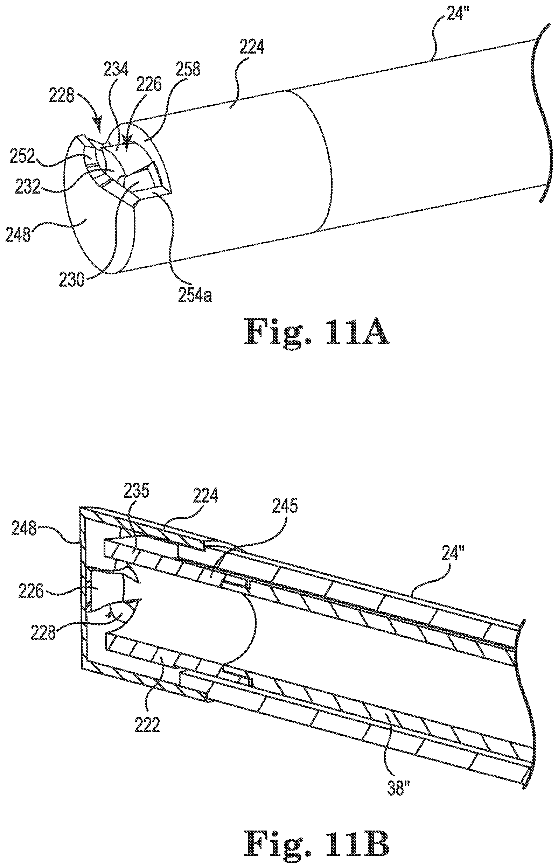

A surgical cutting instrument 10 in accordance with aspects of the present disclosure is shown in FIG. 1. The cutting instrument 10 includes a proximal handpiece 12 and a cutting implement 14 extending distally from the handpiece 12. The cutting implement 14 includes an inner blade assembly 16, an outer blade assembly 18, and a window orientation assembly 20. The inner blade assembly 16 and the outer blade assembly 18 terminate distally at a cutter 26 having a cutting tip and a cutting window, as discussed in more detail below. The inner blade assembly 16 includes an inner blade 22 which has a cutting tip (see also, e.g., FIGS. 6A-6C and 9A-9B). The outer blade assembly 18 includes an outer blade 24 which has a cutting window (see, e.g., FIGS. 7A-7C and 10A-10B). The inner blade 22 is coaxially disposed within the outer blade 24 such that the cutting tip is exposed at the cutting window formed at a distal region of the cutting implement 14 (see, e.g., FIGS. 8A-8B and 11A-11B). Details on the various components are provided below.

The handpiece 12 includes a housing 28 that contains a motor (not shown) for driving the rotational movement of the inner blade assembly 16. The handpiece 12 receives proximal ends of the inner and outer blade assemblies 16, 18 for fluidly connecting internal irrigation and aspiration paths (each not shown) with an irrigation port 30 and an aspiration port 32, respectively, assembled to the housing 28. Regardless, the irrigation path is formed within the housing 28 extending from the irrigation port 30, through the outer blade assembly 18 to the cutting window. The irrigation port 30, in turn, is adapted for fluid connection to tubing (not shown) that is otherwise connected to a fluid source (not shown). Similarly, the aspiration port 32 assembled to the handpiece 12 is in fluid communication with the aspiration path formed within the housing 28 extending from the aspiration port 32, through the inner blade assembly 16 to the cutting tip. The aspiration port 32, in turn, is adapted for fluid connection to tubing (not shown) that is otherwise connected to a vacuum source (not shown) for applying a vacuum to the aspiration path, and thus to the inner blade 22. Additional control of the negative pressure supplied to the cutting tip is provided by an aspiration control hole 34 on the handpiece 12.

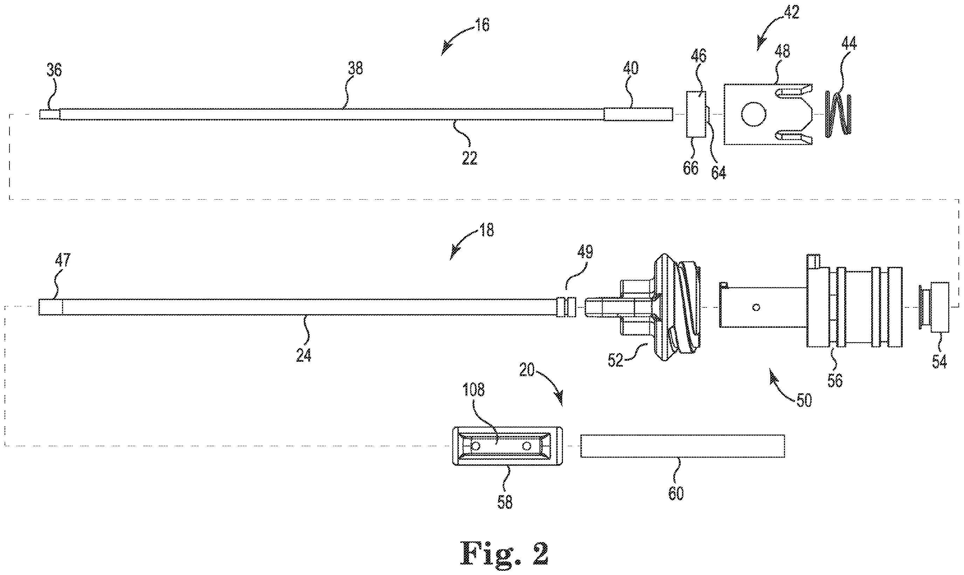

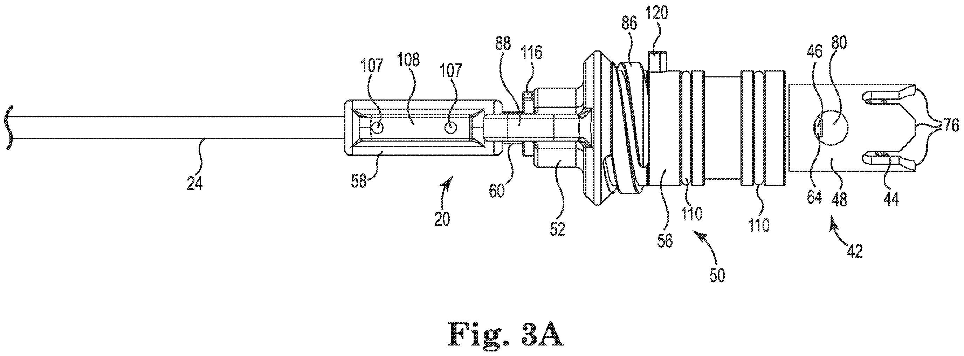

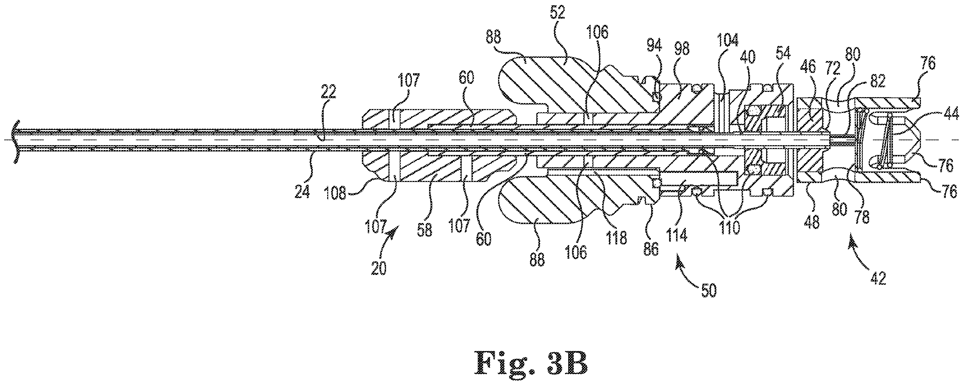

With the above general construction of the cutting instrument 10 in mind, features associated with the cutting implement 14 in accordance with aspects of the present disclosure are shown in greater detail in FIG. 2. The cutting implement 14 includes the inner blade assembly 16 having the inner blade 22 and the outer blade assembly 18 having the outer blade 24. In general terms and with additional reference to FIGS. 3A and 3B, the inner blade 22 is rotatably received within the outer blade 24, with the other components of the inner and outer blade assemblies 16, 18 effectuating connection to the handpiece 12.

As further illustrated in the cross-sectional view of the cutting implement 14 of FIG. 3B, the inner blade 22 defines an aspiration pathway through an interior of the cutting implement 14 to the cutting tip. The inner and outer surfaces of the inner blade 22 are generally smooth and free from burs. The inner blade 22 has a length such that the aspiration pathway may extend continuously through a hub assembly of both the inner blade 22 and the outer blade 24. In particular, the inner blade 22 is coaxially disposed within the outer blade 24 such that a distal end of the outer blade 24 is proximal to a distal end of the inner blade 22.

The inner blade 22 defines a lumen between an open proximal end and a distal end, the distal end having an opening therein communicating with the pathway/lumen and forming a suction inlet through which cut bodily tissue can be aspirated. The inner blade 22 has a distal portion 36, an intermediate portion 38, and a proximal portion 40. In one embodiment, the proximal portion 40 has a slightly larger outer diameter than either the distal portion 36 or the intermediate portion 38. In one embodiment, at least a portion of the proximal end 40 of the inner blade 22 is textured to include a raised fine diamond knurl (not shown). Regardless, the inner blade 22 is an elongated tubular body and terminates at the distal cutting tip is formed in the distal portion 36.

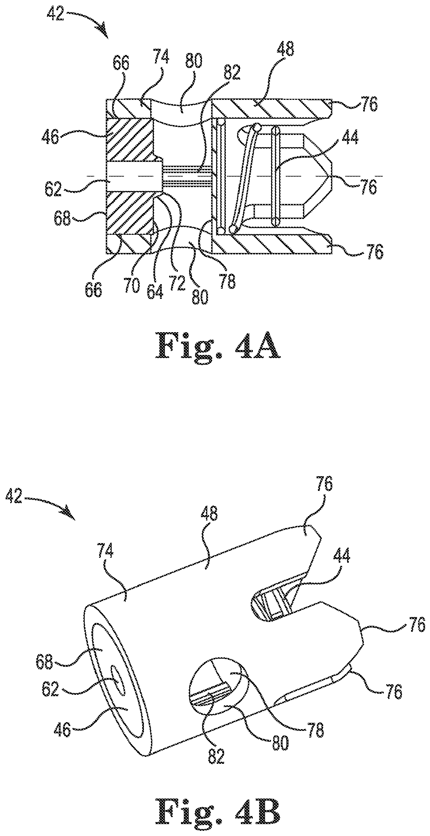

With continued reference also to FIG. 2, the inner blade assembly 16 includes the inner blade 22 as well as an inner hub assembly 42. As described below, the inner hub assembly 42 maintains the inner blade 22 and facilitates connection of the inner blade assembly 16 to the motor (not shown). The inner hub assembly 42 includes a spring 44, an inner hub 46, and a drive hub 48. The inner hub 46 and the drive hub 48 can assume a variety of forms. The inner hub 46 is adhesively, thermally, or otherwise bonded to the drive hub 48. The inner blade 22 extends to the inner hub 46 and can be welded or otherwise bonded to the inner hub 46. The inner blade 22 has an inner surface that defines a lumen. Features associated with one embodiment of the inner hub assembly 42 in accordance with aspects of the present disclosure are shown in greater detail in FIGS. 4A and 4B.

The outer blade 24 is an elongated tubular body defining a central lumen extending between a proximal end and a distal end. The central lumen of the outer blade 24 generally defines uniform inside diameter and is generally uniformly smooth. The outer blade 24 includes a distal region 47 and a proximal region 49. The proximal region 49 includes radially projecting rings spaced apart to accommodate an o-ring disposable in between the radially projecting rings, as discussed more below. With reference to FIG. 3B, when assembled, the inner blade 22 is maintained within the central lumen of the outer blade 24 such that an outer surface of the inner blade 22 and an inner surface of the outer blade 24 define an irrigation pathway to the cutting window. The central lumen of the outer blade 24 is sized to accommodate the inner blade 22 coaxially within and maintain the irrigation pathway between walls of the inner blade 22 and outer blade 24.

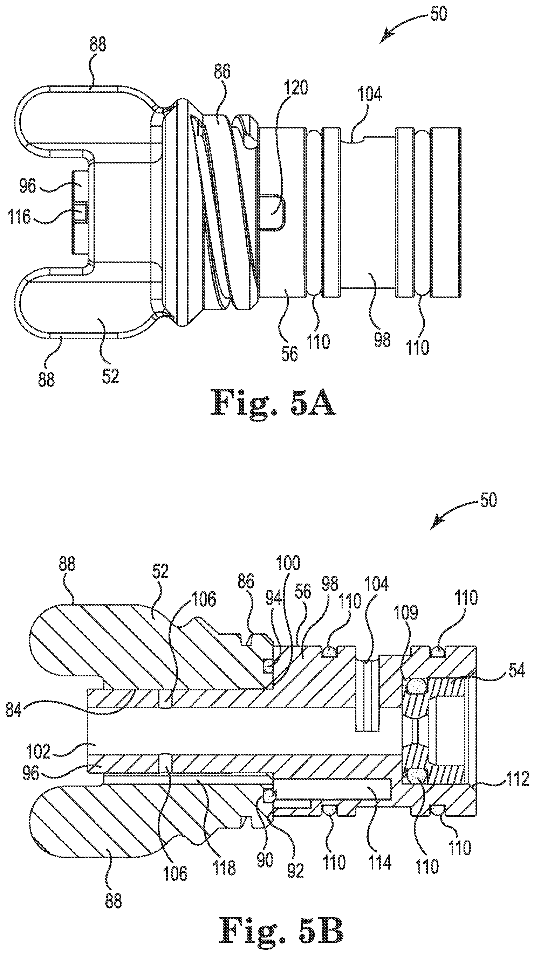

In addition to the outer blade 24, the outer blade assembly 18 includes an outer hub assembly 50 having a fastener 52, a dynamic seal 54, and an outer hub 56. The outer blade 24 extends distally from within the outer hub 56. The fastener 52 removably secures the hub assemblies 42, 50 within the handle 12 (see FIG. 1). The outer hub assembly 50 can assume a wide variety of forms. Features associated with one embodiment of the outer hub assembly 50 in accordance with aspects of the present disclosure are shown in greater detail in FIGS. 5A and 5B.

The window orientation assembly 20 includes a window orientation hub 58 and a tubular shaft 60. The inner diameter of the tubular shaft 60 is sized appropriately to accommodate the outer blade 24. The proximal end of the tubular shaft 60 can have an inner diameter larger than the remainder of the tubular shaft 60 in order to accommodate the radial rings at the proximal region 49 of the outer blade 18 (see, e.g., FIG. 3B). Features associated with one embodiment of the window orientation assembly 20 in accordance with aspects of the present disclosure are shown in greater detail in FIGS. 3B.

With reference to FIGS. 3A and 3B, the inner blade 22 is assembled to the inner hub 46 which in turn is assembled to the drive hub 48 of the inner hub assembly 42. The window orientation assembly 20 is assembled to the outer blade 24 which in turn is assembled to the outer hub assembly 50. The inner blade assembly 16 is assembled with the outer blade assembly 18 and the assembled hubs 42, 50 are coaxially received within the handpiece 12 (not shown), with the outer blade 24 and inner blade 22 extending distal the hub assemblies 42, 50. The inner hub assembly 42 and the outer hub assembly 50 cooperate to facilitate the rotational relationship of the inner blade 22 and the outer blade 24 by the handpiece 12 that supports both the inner blade assembly 16 and the outer blade assembly 18. Rotation of the inner blade 22 is translated to the cutting tip to effect selectively removing the target tissue at the treatment site, as described in greater detail below. With this construction, aspirated liquids and solids (not shown) can be delivered from the cutting tip through the lumen of the inner blade 22 via a sealed pathway. Other constructions capable of effectuating flow of irrigation liquid to the outer blade 24 and aspiration through the inner blade 22 are also envisioned.

The inner hub assembly 42 is shown in greater detail in FIGS. 4A and 4B and includes the spring 44, the inner hub 46, and the drive hub 48. The inner hub 46 includes a central port 62 terminating in a tapered lip 64 and radial side walls 66. The radial side walls 66 of the inner hub 46 extend between a first surface 68 and a second opposing surface 70. The central port 62 is sized and shaped to accommodate the inner blade 22. With additional reference to FIGS. 3A and 3B, the inner blade 22 is insertable into and connectable to the inner hub 46 to provide a fluid connection of the interior of the inner blade 22 with the central port 62 of the inner hub 46 along a longitudinal axis (indicated by the dashed line in FIG. 3B). The tapered lip 64 extends from the second surface 70 to terminate at a rim 72. In one embodiment, the outer surface of the tapered lip 64 extends at a 25° angle from the longitudinal axis (indicated by the dashed line in FIG. 4A) of the central port 62. In one embodiment, the rim 72 of the tapered lip 62 is 0.0254mm to 0.0762mm (0.001" to 0.003") wide around the circumference of the central port 62. In one embodiment, the tapered lip 64 extends 0.254mm (0.010") along the longitudinal axis. Other circumference and height dimensions are also acceptable. The tapered lip 64 effectuates weld joint development between the inner hub 46 and the inner blade 22 (see, e.g., FIG. 3B).

The drive hub 48 of the inner hub assembly 42 includes perimeter walls 74 terminating at one end in prongs 76. The prongs 76 are configured to facilitate connection with the motor housed in the handpiece 12. The drive hub 48 has an open proximal end and an open distal end. A stop 78 extends generally perpendicular to the perimeter walls 74 between the proximal end and the distal end. The stop 78 can be planar and extend to fully separate the spring 44 from the central port 62. The stop 78 is formed between side ports 80 and the prongs 76 along the longitudinal axis (indicated by the dashed line in FIG. 4A). The stop 78 provides a distal stop for springs. The distal end of the drive hub 48 is configured to receive the inner hub 46 and in some cases terminates at a planar surface. The perimeter walls 74 define a cylindrical body. The side ports 80 are formed in the perimeter walls 74. In one embodiment, the perimeter walls 74 include two diametrically opposed side ports 80. The side ports 80 of the drive hub 48 are configured to fluidly connect with the central port 62 of the inner hub 46.

With further reference to FIGS. 4A and 4B, a length of the radial side walls 66 along the longitudinal axis between the first surface 68 and the second opposing surface 70 is such that it does not infer with fluid flow of drive ports 74 of the drive hub 48. The walls 66 are configured for insertion within the perimeter walls 74 of the drive hub 48 and have an outer diameter slightly less than an inner diameter of the perimeter walls 74. In one embodiment, the rim 72 extends fully within the drive hub 48. The radial side walls 66 fit tightly within the drive hub 48 and form a fluid tight seal against the perimeter walls 74. The drive hub 48 includes inner hub stops 82 that extend from the stop 78 to a predetermined distance along the inner surface of the perimeter walls 74. The predetermined distance is a distance suitable to allow the inner hub 46 to be inserted and the first surface 68 positioned flush with, or on the same plane as, a distal end surface of the drive hub 48. As assembled, the inserted inner hub 46 is clear of, and does not block, fluid communication of the side ports 80. In one embodiment, the inner hub stops 82 are a length equal to, or greater than, a diameter of the side ports 80. The inner hub stops 82 extend across the diameter of the side ports 80 on the inner surface of the drive hub 48 radially adjacent to the side ports 80. The inner hub stops 82 projects interiorly from the inner surface of the perimeter walls 74 sufficiently to prevent the inner hub 46 from being inserted into the drive hub 48 a distance that would partially or fully block the side ports 80. The stop 78 extends across a diameter of the drive hub 48 proximal to the side ports 80 opposing the inner hub 46. In other words, when assembled, the plate 78 and the drive hub 48 are disposed on opposite sides of the side ports 80.

The outer hub assembly 50 is shown in greater detail in FIGS. 5A through 5B and includes the fastener 52, the dynamic seal 54, and the outer hub 56. The fastener 52 includes an open core 84 extending longitudinally through the fastener 52 and open at both proximal and distal ends. The core 84 is sized and shaped for a distal portion of the outer hub 56 to extend through, as discussed further below.

The fastener 52 includes threads 86 on an exterior surface to removably secure the outer hub assembly 50 within the handpiece 12 (i.e., threads 86 are mate-able with threads in the handpiece, not shown). The fastener 52 includes winged tabs 88 extending distally away from the threads 86 as well as radially outward. The winged tabs 88 are configured for ease of handling and turning the threads 86 of the fastener 52 to engage or disengage the cutting implement 14 to or from the handpiece 12 (see FIG. 1). Other engagement and disengagement mechanisms are also acceptable. In one embodiment, two winged tabs 88 are included and are disposed on opposing sides of the fastener 52. Other quantities and configurations of winged tabs 88 can also be suitable. In one embodiment, threads 86 are provided to allow the fastener 52 to be turned 180° when securing within the handpiece 12. Sufficient threads 86 are included to prevent the fastener 52 from inadvertently disengaging from the handpiece 12 when the surgical cutting instrument 10 is in use. In any regard, suitable threads 86 are provided to secure the outer hub assembly 50, and the cutting implement 14, to the handpiece 12 until a user rotates the fastener 52 by rotationally pushing against the winged tabs 88 to disengage the threads 86 from the handpiece 12. In one embodiment, a circumferential notch 90 is included at a proximal end 92. An o-ring 94 is insertable into the circumferential notch 90. The o-ring 94 is disposed against to the outer hub 56 when assembled and can reduce or absorb some of the vibrations, particularly in an axial direction, of the cutting implement 14 during operation.

The outer hub 56 includes a neck 96 and a base 98. A radial shoulder 100 is defined between the neck 96 and the base 98 and radially extends from an outer diameter of the neck 90 to an outer diameter of the base 98. The neck 96 is sized and configured to extend within and through the fastener 56. A passageway 102 extends through the neck 96 and the base 98. The irrigation inlet 104 extends from an exterior surface of the outer hub 56 and fluidly connects with the passageway 102. In one embodiment, the passageway 102 extends along the longitudinal axis (indicated by the dashed line in FIG. 5B) and the irrigation inlet 104 extends perpendicularly to the exterior surface of the outer hub 56. In accordance with aspects of the disclosure, the irrigation inlet 104 is disposed within the handpiece 12 to fluidly connect to the fluid pathway and irrigation port 32 when assembled to the handpiece 12.

With additional reference to FIG. 3B, the outer blade 24 extends within the passageway 102 and terminates distal to the irrigation inlet 104 such that the irrigation inlet 104 freely communicates with the lumen of the outer blade 24. The window orientation assembly 20 extends distally from within the outer hub assembly 42. In particular, the tubular shaft 60 extends coaxially around the outer blade 24 and terminates within the outer hub 56. A proximal end of the tubular shaft 60 terminates approximately flush with the proximal end of the proximal region 49 of the outer blade 24. A seal ring 110 is disposed between the radial rings formed on the proximal region 49 of the outer blade 24 to facilitate a fluid tight seal between the proximal region 49 of the outer blade 24 and the tubular shaft 60. The tubular shaft 60 can be adhesively attached within the outer hub 56. At least one glue weep port 106 extends at an angle to, and in some cases perpendicular, to the longitudinal axis extending along a length of the passageway 102. The at least one adhesive weep port 106 directly and fluidly connects to the passageway 102 and is also fluidly open at an exterior surface of the outer hub 56. Adhesive (not shown), when used to adhere the outer blade 24 and, in some embodiments, the tubular shaft 60, within the passageway 102 of the outer hub 56, is inserted in the distal end after or with insertion of the outer blade 24 and excess adhesive can exit the neck 96 at least one adhesive weep port 106 during assembly.

With continued reference to FIG. 3B, the distal end of the tubular shaft 60 terminates within the orientation hub 58. The orientation hub 58 is disposed along the outer blade 24 distal to the outer hub assembly 50. The orientation hub 58 can be adhesively or otherwise attached to the outer blade 24 and the tubular shaft 60. At least one adhesive weep port 107 extends at an angle to, and in some cases perpendicular, to the outer blade 24 and the tubular shaft 60. The at least one adhesive weep port 107 is fluidly open at an exterior surface of the orientation hub 58. Adhesive (not shown), when used to adhere the outer blade 24 and the tubular shaft 60 within the orientation hub 58, is inserted after or with insertion of the outer blade 24 and the orientation hub 58 and excess adhesive can exit the glue weep ports 107 during assembly. The orientation hub 58 is generally tubular and includes an arm 108 projecting radially. When assembled, the arm 108 of the orientation hub 58 is aligned with the cutting window of the outer blade 24 in order that the user can selectively rotate and realign the arm 108, and consequently, the cutting window to face and be open toward the desired tissue to be removed.

With continued reference to FIGS. 3B and 5B, the inner blade 22 extends beyond the proximal end of the outer blade 24 and through the dynamic seal 54 disposed in a bore 109 at the proximal end portion of the outer hub 56 to connect with the inner hub assembly 44. The dynamic seal 54 fluidly seals around the inner blade 22. In addition, as described in greater detail below, the outer surface of the outer hub 56 is adapted to receive seal rings 110 (e.g., o-rings) on either side (i.e., proximal and distal) of an irrigation inlet 104 to effectuate a fluid-tight seal between the outer hub 56 and the handpiece 12 (not shown). As assembled, the seal rings 110 can also provide radial vibration attenuation, thereby reducing vibration at the cutter 26. In some embodiments, the dynamic seal 54 and the seal rings 110 are a polytetrafluoroethylene (PTFE) material such as Teflon^{®}, although other suitable materials are also acceptable.

In one embodiment, as illustrated in FIG. 5B, the proximal end of outer hub 56 includes bevel 112 to provide additional clearance to allow for axial adjustment of the inner hub assembly 42 with respect to the outer hub assembly 50 when adjoined. The bevel 112 of the outer hub 56 enables axial adjustment of the inner hub assembly 42 to allow for seal compression without imparting pre-load to DCM thrust surfaces.

In one embodiment, the outer hub 56 includes an identification port 114. The identification port 114 is configured to accept a radio-frequency identification device (RFID). The RFID includes data to identify the size and blade type, for example, of the cutting implement 14 that is transferred to an integrated power-console (IPC) when the cutting implement 14 is assembled with the handpiece 12. The IPC, upon receiving information from the RFID, can supply power to operate the cutting implement 14 at a suitable speed as well as fluid and aspiration suitable for the specific cutting implement 14. Other cutting implement identification systems, such as magnetic hall sensors, for example, are also acceptable.

With continued reference to FIGS. 5A and 5B, the outer hub 56 can include a protrusion 116 at the distal end of the neck 96. The protrusion 116 is configured to align with an alignment slot 118 in the fastener 52. The protrusion 116, when rotated and disposed within alignment slot 118, couples the fastener 52 and the outer hub 56 together. Additionally, a tab 120 disposed on the base 98 extends outwardly and is aligned radially with the protrusion 116 on the neck 96. The tab 120 is configured to slidably mate within a slot in a collar of the handpiece 12 (not shown). When assembled, the tab 120 prevents the cutting implement 14 from rotating when in use and provides a reference point to the cutting window orientation.

With reference to FIGS. 5A and 5B, the dynamic seal 54 can be provided to effectuate a fluid-tight seal between the inner blade 22 and the outer blade 24. The dynamic seal 54 can be maintained within the bore 109 at the distal end of the outer hub 56 to seal around the inner blade 22 extending through the outer hub 56. An o-ring 110 can also be disposed in the bore 109 with the dynamic seal. The dynamic seal 54 is configured to fluidly seal around the proximal portion 40 of the inner blade 22, as illustrated in FIG. 3B. With this construction, an irrigation liquid (not shown) to the outer blade 24 can be delivered to the lumen of the outer blade 24 via a sealed pathway. The inner blade 22 extends through the dynamic seal 54 to fluidly seal within the inner hub 48 of the inner hub assembly 42. With this construction, aspirated liquids and solids (not shown) can be delivered from the cutting tip through the lumen of the inner blade 22 via a sealed pathway. Other constructions capable of effectuating flow of irrigation liquid to the outer blade 24 and aspiration to the inner blade 22 are also envisioned.

FIGS. 8A and 8B are enlarged cross-sectional and perspective views of an assembled distal portion 122 of an inner blade 22' are illustrated in FIGS. 6A through 6C and the distal region 124 of an outer blade 24' is illustrated in FIGS. 7A through 7C of a cutting implement according to one embodiment of the present disclosure. As illustrated, upon final assembly, a cutting tip 126 provided at the distal portion 122 of the inner blade 22' is selectively exposed at a cutting window 128 on the distal region 124 of the outer blade 24'. Aspects of the inner blade 22' and outer blade 24' are similar to the inner blade 22 and outer blade 24, respectively.

With reference to the distal portion 122 of the inner blade 22' illustrated in FIGS. 6A through 6C, the cutting tip 126 includes an open distal end having cutting surfaces formed by radial and longitudinal projections and recesses. An end notch 130 extends between projections 132. In general terms, the projections 132 form the distal end cutting surface of the cutting tip 126. In one embodiment, two opposing side projections 132 at least partially defined by a U-shaped or semi-circular notch 130 formed between the projections 132. The distal most end of the cutting tip 126 is defined by end edges 134 formed at a distal end of the projections 132. The end edges 134 are radially planar in a plane perpendicular to the longitudinal axis. The end edges 134 are beveled in a longitudinally inward direction to form a sharpened cutting edge. In one embodiment, the end edges 134 are beveled at a 45° angle.

The cutting tip 126 includes teeth 136a, 136b projecting along opposing radial side edges 138 of a central opening 140 of the cutting tip 126. The teeth 136a, 136b are oriented in opposing pairs that are longitudinally aligned to extend toward each other across the opening 140 of the cutting tip 126 and generally perpendicular to end edges 134 and the projections 132. Edge surfaces of the teeth 136a, 136b are beveled, or angled, towards the interior of the inner blade 22'. The teeth 136a are formed on the projections 132 such that tips 142a are aligned with the end edges 134. At least one pair of teeth 136b is included along the side edges 138 of the central opening 140. The teeth 134a, 134b can have different heights. Valleys 144 are formed between tips 142a and 142b of teeth 136a, 136b. The teeth 136b are formed to have an angle "X".

As best illustrated in FIG. 6B, the distal portion 122 has a first outer diameter d₁ slightly larger, or greater, than a second outer diameter d₂ of an intermediate portion 38'. The intermediate portion 38' is similar to intermediate portion 38 described above. In one embodiment, the distal portion 122 is laser welded to the intermediate portion 38'. According to the main embodiment of the invention, an irrigation channel 146 extends from the cutting tip 126 formed on the distal portion 122 to a distal end of the intermediate portion 38'. The irrigation pathway extends along the irrigation channel 146 to the cutting tip 126, as described in more detail below.

As discussed above, the first outer diameter d₁ of the distal portion 122 is slightly larger, or greater, than the second outer diameter d₂ of the main portion 38'. For example, in one embodiment, the outer diameter d₁ of the distal portion 122 is 1.5621mm to 1.5748mm (0.0615 inches to 0.0620 inches) and the outer diameter d₂ of the intermediate portion 38' is 1.4732mm to 0.0254mm (0.058" +/- 0.001 inches). In order maintain irrigation flow between the inner blade 22' and the outer blades 24', the irrigation channel 146 is formed along the distal portion 122 of the inner blade 22'. In one embodiment, the irrigation channel 146 is a portion of the outer surface that is planar and is recessed to extend within the thickness of the inner blade 22' between the inside surface and the outside surface. In one embodiment, the irrigation channel 146 is centered between the side edges 138 to be fluidly open at the central opening 140 of the cutting tip 126. The irrigation channel 146 extends from the central opening 140 of the cutting tip 126, along the distal portion 122, to the main portion 38' of the inner blade 22'. In one embodiment, the distal portion 122 has a length of 5.08mm (0.200 inches) and the opening 140 extends approximately 1.016mm (0.040 inches) along the longitudinal axis and the distal portion 122.

With reference to FIGS. 7A through 7C, the cutting window 128 is formed at the distal region 124 of the outer blade 24' is defined by shearing edges on the tubular sides and an end cap 148 of the outer blade 24'. The outer blade 24' is similar to outer blade 24 described above. The end cap 148 can be shaped as a segment or a circular zone, for example, at the distal end of the tubular body of the outer blade 24'. In this manner, both end cutting and side cutting, or shearing, surfaces are provided. The geometry of the cutting window 128 is configured to avoid clogging. For example, the cutting window 128 can have a length equivalent to the inner diameter of the inner blade 22' in order to avoid cutting off pieces of tissue large enough to clog the inner blade 22' and disrupt cutting. The outer surface of the distal region 124 can include markings 150 to visually indicate a depth of the cutting tip 126, and thus the cut, with respect to the tissue. For example, the markings 150 could be placed to indicate a cutting depth of 0.5 millimeter, 1.0 millimeter, 1.5 millimeters, etc. with lines and/or numbers.

In one embodiment, the end cap 148 is planar and the outer blade 24' is cylindrical rather than hemispherical at the distal region 124. The planar end cap 148 is perpendicular to cylindrical side walls of the distal region 124. The end cap 148 is joined to the tubular side walls of the distal region 124 of the outer blade 24' to squared off interior surfaces along the perimeter intersection of the end cap 148 and the side walls of outer blade 24'. In other words, the interior of the distal region 124 of the outer blade 24' at the intersection of the end cap 148 and the tubular side walls of the outer blade 24' forms a right angle (i.e., 90°), without a radius. The squared off surfaces of the end cap 148 and side walls expose a maximum surface area to the cutting end edges 134 of the projections 132 of the cutting tip 126 (see, e.g., FIG. 8A). Due to the increased cutting surface area, cutting of the tissue occurs more quickly as compared to that of a hemispherical end cutter or a cavitational ultrasonic surgical aspirator (CUSA).

With continued reference to FIGS. 7A through 7C, an end window edge 152 is formed on the end cap 148. In one embodiment, the end window edge 152 is positioned such that the resulting opening is less than half of the end cap 148. In one embodiment, the end window edge 152 is linear, extending across a width of the outer blade 24' to define an open semicircular shape on the end cap 148. The end window edge 152 is beveled, or angled, towards an exterior of the outer blade 24'.

Side window edges 154 extend from the end window edge 152 along the tubular sides of the distal region 126. The side window edges 154 are serrated to include window teeth 156. The window teeth 156 are disposed on opposing radial sides of the cutting window 128. Similar to the teeth 136a, 136b on the inner blade 22', the window teeth 156 are oriented in opposing pairs that are longitudinally aligned to extend toward each other across the cutting window 128 and extend generally perpendicular to the end cap 148. The cutting window 128 includes outwardly beveled edges. For example, the side window edges 154, including edge surfaces of the window teeth 156, are beveled, or angled, towards the exterior of the outer blade 24'. In one embodiment, an inner portion of the side window edges 154 is sharpened at an angle different from the outer portion of the side window edges 154. A suitable quantity of window teeth 156 are included on the cutting window 128 to correspond with, and interact with, the quantity of teeth 136 on the cutting tip 126. In one embodiment, a single pair of window teeth 154 and a single pair of teeth 136b are provided. An angle "Y" formed by the sides of the window teeth 134 complements the angle "X" of the teeth 136b of the cutting tip 126. For example, when the angle "Y" of the window teeth 156 is 57° and the angle "X" of the teeth 136b is 46°. Regardless, the tips of the window teeth 156 are longitudinally offset from the tips 142a, 142b.

The cutting window 128 includes a top window edge 158 extending between the side window edges 154, opposite the end window edge 152, to form the cutting window 128. The top window edge 158 includes a center section 160 and opposing recesses 162 to form additional shearing surfaces. As best illustrated by FIGS. 7A and 7B, the center section 160 can be slightly angled and the recesses 162 are angled distinctly from both the adjacent side window edges 154 and the center section 160 to create additional shearing, or cutting, surface.

Upon final assembly, as best shown in FIGS. 8A and 8B, the cutting tip 126 is positioned at the cutting window 128 with the two components being rotatable relative to one another as discussed above. The cutting tip 126 includes surfaces or edges, including those formed by protrusions 132 and teeth 136a, 136b, for engaging tissue via the cutting window 128 in the distal end of the outer blade 24'. The edges forming the cutting tip 126 are beveled inwardly and the edges defining the cutting window 128 are generally beveled outwardly to form cooperating shearing surfaces. As the inner blade 22' is rotatably driven at its proximal end, for example by the motorized handpiece 12, the surface or edge of the cutting tip 126 will cooperate with the cutting window 128 in the outer blade 24' to shear, cut, or shave the tissue. The end edges 134 of the cutting tip 126 directly contact the end cap 148 of the outer blade 22' to provide end cutting. The spring 44 of the inner hub assembly 42 biases the end edges 134 toward the end cap 148. In general terms, the inner geometry of the cutting tip 126 is designed to impale the tissue as the cutting tip 126 is rotated and provides both end cutting and side cutting via cylindrical geometry. As discussed further below, peripheral edges of the cutting tip 126 opening formed are positioned adjacent cutting window 128 such that the cutting edges of the cutting tip 126 can engage bodily tissue through the cutting window 128 and pull the tissue against the edges defining the cutting window 128 to shear the tissue. Micro-machining, or other suitable methods, can be used to create the profile and the sharp angular and edges of the cutting tip 126 and cutting window 128.

As discussed above, the distal portion 122 of the inner blade 22' has a diameter d₁ that is larger than the diameter d₂ of the main portion 38' of the inner blade 22'. For example, the outer diameter d₁ of the distal portion 122 can be 1.5748mm (0.0620 inches) and the outer diameter d₂ of the main portion 38' can be 1.4732mm +/- 0.0254mm (0.058" +/- 0.001 inches). The clearance between the outer surface of the distal portion 122 of the inner blade 22' and the inner surface of the outer blade 24' can be 0.0508mm (0.002 inches) or less, and in particular, the clearance at the cutting window 128 can be 0.00236mm to 0.027686mm (0.000093 inches to 0.00109 inches). As assembled, the outside surface of the inner blade 22' is coated with a biocompatible tungsten-carbide/carbon coating to prevent galling and decrease friction between the inner and outer blades 22', 24'. The irrigation channel 146 extending along the distal portion 122 of the inner blade 22' and is fluidly open with the cutting tip 126 in order to maintain irrigation to the cutting tip 126 from between the inner and outer blades 22', 24' and to accommodate the increased diameter of the distal portion 122 of the inner blade 22' within the outer blade 24'. With the clearance of 0.00236mm to 0.027686mm (0.000093 inches to 0.00109 inches) at the cutting window 128, a shearing of the fibrous tissue between the moving inner blade 22' and the stationary outer blade 24' can occur and tissue is not dragged between the inner and outer blades 22', 24' and to be eventually torn by multiple rotations of the inner blade 22'.

The inner and outer blades 22', 24' can be manufactured of a metal, such as stainless steel, or other hard material suitable for use in surgery. The distal portion 122 of the inner blade 22' is fabricated separately from the main portion 38' and the distal region 124 is fabricated separately from the remainder of the outer blade 24'. The distal region 124 having the cutting window 128 can be formed of a material different from, and having a Rockwell hardness greater than, the remainder of the outer blade 24'. For example, the distal region 124, including the end cap 148, can be formed of 440C stainless steel (s.s.) with a hardness above 50 HRC and the remainder of the outer blade 24' can be formed of 304L s.s. tubing. Similarly, the distal portion 122 having the cutting tip 126 can be formed of a material different from, and having a Rockwell hardness greater than, the remainder of the inner blade 22'. For example, the distal portion 122 can be formed of 440C s.s. with a hardness above 50 HRC and the remainder of the inner blade 22' can be formed of 304L s.s. tubing. Micro-machining such as electrical discharge machining (EDM), electrochemical machining (ECM), mechanical machining, chemical machining, micro-electro-mechanical system (MEMS) processing, or other suitable micro-machining methods can be used to form the cutting inner and outer blades, in particular, the cutting tip 126 and cutting window 128. In one embodiment, the distal region 124 is laser beam welded to the remainder of the outer blade 24' and the distal portion 122 is laser beam welded to the main portion 38' of the inner blade 22'.

In accordance with principles of the present disclosure, an embodiment of a distal end of a cutting implement is illustrated in FIGS. 11A and 11B. FIGS. 9A and 9B are enlarged cross-sectional and perspective views of the distal portion 222 of the inner blade 22" of FIGS. 11A and 11B and the distal region 224 of the outer blade 24" is illustrated in FIGS. 10A and 10B. As illustrated, upon final assembly, a cutting tip 226 provided at the distal portion 222 of the inner blade 22" is selectively exposed at a cutting window 228 on the distal region 224 of the outer blade 24". Aspects of the inner blade 22" and outer blade 24" are similar to the inner blade 22, 22' and outer blade 24, 22', respectively.

With reference to the distal portion 222 illustrated in FIGS. 9A and 9B, the cutting tip 226 includes openings 230 defined by cutting surfaces of the cutting tip 226. The cutting tip 226 includes a terminal end portion 232 and opposing side portions 234. The side portions 234 extend distally, or along a longitudinal axis of the distal portion 222, to connect the terminal end portion 232 with the body of the distal portion 222. Openings 230 are defined by the cutting surfaces of the terminal end portion 232 and side portions 234 and provide side and end cutting and fluid pathways. As illustrated in FIG. 9A, a double-edged cutting surface of the cutting tip 226 is formed.

An outer surface of the side portions 234 is arcuate and maintains an outer circumference of the first section 235 of the distal portion 222. The terminal end portion 232 has a top surface 236 defined by curvilinear side edges 238 extending between opposing end edges 240. In one embodiment, an interior surface opposite the top surface 236 of the terminal end portion 232 is also planar. The top surface 236 intersects with the side portions 234 to form the arcuately shaped end edges 240. In one embodiment, the curvilinear side edges 238 of the top surface 236 are symmetrical about a "y" axis formed along the planar top surface 236, and perpendicular to the longitudinal axis, or "x" axis, of the inner blade 22". In one embodiment, each of the side edges 238 includes a semicircular center portion and opposing concave portions. In other words, the concave portions of the side edges 238 extend between the center portions and the end edges 240. In one embodiment, the concave portions joined on opposing ends of the center portion of one side edge 238 have the same radii, although differing radii can also be acceptable.

Sides 242 of the side portions 234 are tapered, or angled, curvilinearly inward from the intersecting planar top surface 236 to an opening surface 244 to form sharpened cutting edges. In one embodiment, the opening surface 244 is planar and parallel to the top surface 236. As illustrated best in FIG. 9A, the tapered sides 242 of the side portions 234 are smooth and without intermediate projections or teeth. In one embodiment, the terminal end portion 232 has a thickness of 0.127mm +/- 0.0127mm (0.0050" +/- 0.0005 inches) between the top surface and the opposing surface and the top surface 236 of the cutting tip 226 is positioned 0.508mm +/-0.0254mm (0.020" +/- 0.001 inches) from the opening surface 244 to form a longitudinal length of the openings 230. Openings 230 are fluidly open to the central lumen of the inner blade 22" as described further below.

As best illustrated in FIG. 9B, the distal portion 222 includes the first section 235 having a first outer diameter d₃ slightly larger, or greater, than a second outer diameter d₄ of a second section 245. For example, in one embodiment, the outer diameter d₃ of the first section 235 is 1.5748mm (0.0620 inches) and the outer diameter d₄ of the second section 245 is 1.4732mm +/-0.0254mm (0.058" +/- 0.001 inches).

In order maintain irrigation flow between the inner blade 22" and the outer blades 24", the irrigation channel 246 is formed along the first section 235 of the distal portion 222 of the inner blade 22". The irrigation pathway extends along the irrigation channel 246 to the cutting tip 226, as described in more detail below. In the main embodiment of the invention, the irrigation channel 246 is a portion of the outer surface that is planar and is recessed to extend within the thickness of the inner blade 22" (i.e., between the inside surface and the outside surface). In one embodiment, the irrigation channel 246 has slightly chamfered longitudinal edges. In one embodiment, there are two opposing irrigation channels 246, one each centered between the side portions 234 of the cutting tip 226 and each fluidly open at the openings 230 of the cutting tip 226. The irrigation channels 246 extend from the openings 230 of the cutting tip 226, along the first section 235, to the second section 245 of the inner blade 22". In one embodiment, the first section 235 has a length of 1.524mm (0.060 inches) along the "y", or longitudinal, axis.

With additional reference to FIG. 11B and similar to the previous embodiments, the distal portion 222 can be formed separately from, and joined together with, an intermediate portion 38". In one embodiment, the second section 245 has an increased proximal inner diameter suitable to accommodate an outer diameter of the intermediate portion 38" extendable within the distal portion to a predetermined distance. In one embodiment, a lip of the intermediate portion 38" extends within the second section 245 and distal portion 222 is laser welded to the main portion 38". In one embodiment, the proximal inner diameter of the second section 245 is 12.7mm +/-0.0127mm (0.500" +/- 0.0005 inches) while the remainder of the distal portion 222 has an inner diameter of 1.0668mm +/- 0.0254mm (0.042" +/- 0.001 inches).

With reference to FIGS. 10A and 10B, the cutting window 228 formed at the distal region 224 of the outer blade 24" is defined by shearing edges on the tubular sides and an end cap 248 of the outer blade 24". In this manner, both end cutting and side cutting, or shearing, surfaces are provided. The geometry of the cutting window 228 is sized and shaped to avoid clogging and maintain a continuously open fluid pathway between the exterior and the interior central lumen of the inner blade 22". For example, the cutting window 228 can have a length along the longitudinal axis approximately equivalent, or slightly larger than, the distance between the top surface 236 and the opening surface 244 of the cutting tip 226 in order to avoid cutting off pieces of tissue large enough to clog the inner blade 22" and disrupt cutting. The outer surface of the distal region 224 can include markings 250 to visually indicate a depth of the cutting tip 226, and thus the cut, with respect to the tissue. For example, the markings 250 could be placed to indicate a cutting depth of 0.5 millimeter, 1.0 millimeter, 1.5 millimeters, etc. with lines and/or numbers.

The end cap 248 is planar and the outer blade 24" is cylindrical rather than hemispherical at the distal region 224. An interior surface of the tubular side wall and the end cap 248 of the distal region 224 are smooth. The planar end cap 248 is perpendicular to cylindrical side walls of the distal region 224. The end cap 248 is joined to the tubular side walls of the distal region 224 of the outer blade 24" with squared off interior surfaces along the perimeter intersection of the end cap 248 and the side walls of outer blade 24". In other words, the interior of the distal region 224 of the outer blade 24" at the intersection of the end cap 248 and the tubular side walls of the outer blade 24" forms a right angle (i.e., 90°), without a radius. The squared off surfaces expose a maximum surface area to the cutting end edges 234 of the cutting tip 226. Cutting of the tissue occurs more quickly in the present embodiments as compared to that of a hemispherical end cutter or a CUSA due to the increased cutting surface area.

With continued reference to FIGS. 10A and 10B, an end window edge 252 is formed on the end cap 248 to partially define the open shape of the cutting window 228 on the end cap 248. In one embodiment, the end window edge 252 is positioned such that the resulting opening is less than half of the end cap 248. In one embodiment, the end window edge 252 is generally non-linear, extending across a width of the outer blade 24". The end window edge 252 is curved and a varying surface area of the cutting tip 226 is exposed at the cutting window 228 as the cutting tip 226 rotatably passes through the cutting window 228. In one embodiment, two cutting windows 228 are included (not shown). As best illustrated in FIG. 11A, the end window edge 252 is beveled, or angled, towards an exterior of the outer blade 24".

Side window edges 254a, 254b extend from the end window edge 252 along the tubular sides of the distal region 224 of the outer blade 24". The side window edges 254a, 254b can extend from the interior surface to an exterior surface of the tubular side walls flatly, or squarely (i.e., without beveled edges) or can be beveled outwardly. In one embodiment, as illustrated in FIG. 10A, the side window edge 254a extends parallel to the longitudinal axis of the outer blade 24". In one embodiment, the side window edge 254b extends from the end cap 248 at an acute angle that decreases the surface area of the tubular side walls as the side window edge 254b extends away from the end cap 248. In another embodiment, the side window edge 254b extends from the end cap 248 parallel to the longitudinal axis for a predetermined distance and then extends at an acute angle that decreases the surface area of the tubular side walls as the side window edge 254b extends away from the end cap 248.

The cutting window 228 includes a top window edge 258 extending between the side window edges 254, opposite the end window edge 252, to form the cutting window 228. In one embodiment, the top window edge 258 extends along a partial circumference of the tubular body in a plane parallel to the end cap 248. The end window edge 252, the side window edges 254a, 254b, and the top window edge 258 all form shearing surfaces defining the cutting window 228.

The distal region 224 has an outer diameter that is constant, or same. The interior of the distal region 224 has a first inner diameter d₅ and a second inner diameter d₆. The second inner diameter d₆ is greater than the first inner diameter d₅. The second inner diameter d₆ of the distal region 224 is sized and shaped to accommodate a lip of the outer blade 24".

Upon final assembly, as best shown in FIGS. 11A and 11B, the cutting tip 226 is positioned at the cutting window 228 with the two components being rotatable relative to one another as discussed above. As the inner blade 22" is rotatably driven at its proximal end, for example by the motorized handpiece 12, the surfaces or edges of the cutting tip 226 of the inner blade 22" will cooperate with the cutting window 228 in the outer blade 24" to shear, cut, or shave the tissue. In general terms, the inner geometry of the cutting tip 226 is designed to impale the tissue as the cutting tip 226 is rotated and provides both end cutting and side cutting via cylindrical geometry and end and side openings of the cutting window 228 and cutting tip 226. The end edges 234 of the cutting tip 226 directly contact the end cap 248 of the outer blade 22" to provide end cutting. The spring 44 of the inner hub assembly 42 biases the end edges 234 toward the end cap 248. The direction of the cutting tip 226 rotation is toward the side window edge 254b, and thus, away from side window edge 254a. In this manner, the tissue impaled by the cutting tip 226 is rotated toward side window edge 254b where it is sheared off. As discussed further below, peripheral edges of the cutting tip 226 are positioned adjacent cutting window 228 such that the cutting edges of the cutting tip 226 can engage bodily tissue through the cutting window 228 and pull the tissue against the edges defining the cutting window 228 to shear the tissue. Micro-machining, such as ECM or EDM, for example, can be used to create the profile and the sharp angular and beveled edges of the cutting tip 226 and cutting window 228.

The outside surface of the inner blade 22", or the inside surface of the outer blade 24", is coated with a biocompatible tungsten-carbide/carbon coating to prevent galling and decrease friction between the inner and outer blades 22", 24". The irrigation channel 246 extends along the first section 235 of the inner blade 22" and is fluidly open with the cutting tip 226 in order to maintain irrigation to the cutting tip 226 from between the inner and outer blades 22", 24" and to accommodate the increased diameter of the first section 235 of the inner blade 22" within the outer blade 24". With the clearance of 0.00236mm to 0.027686mm (0.000093 inches to 0.00109 inches) between the cutting tip 226 and the cutting window 228, a shearing of the fibrous tissue between the moving inner blade 22" and the stationary outer blade 24" can occur and tissue is not dragged between the inner and outer blades 22", 24". Embodiments of the cutting device, in accordance with aspects of the present disclosure, are suitable for cutting tumors ranging from the very soft to the very fibrous. The flat, or planar, shaped distal cutting ends of the inner and outer blades are suitable for digging into and impaling tissue that is not likely to deflect under vacuum pressure.

As with the previous embodiments, the inner and outer blades 22", 24" can be manufactured of a metal, such as stainless steel, or other hard material suitable for use in surgery. The distal portion 222 of the inner blade 22" is monolithically fabricated of a single piece of material and subsequently coupled to the intermediate portion 38". Similarly, the distal region 224 is monolithically fabricated of a single piece of material and subsequently coupled to the outer blade 24". The distal region 224 having the cutting window 228 can be formed of a material different from, and having a Rockwell hardness greater than, the remainder of the outer blade 24". For example, the distal region 224, including the end cap 248, can be formed of 440C stainless steel (s.s.) with a hardness above 50 HRC and the remainder of the outer blade 24" can be formed of 304L s.s. tubing. Similarly, the distal portion 222 having the cutting tip 226 can be formed of a material different from, and having a Rockwell hardness greater than, the remainder of the inner blade 22". For example, the distal portion 222 can be formed of 440C s.s. with a hardness above 50 HRC and the remainder of the inner blade 22" can be formed of 304L s.s. tubing. In one embodiment, the distal region 224 is laser beam welded to the remainder of the outer blade 24" and the distal portion 122 is laser beam welded to the main portion 38' of the inner blade 22".

The system 10 is highly useful in the surgical treatment (i.e., removal) of intracranial and spinal tumors (as well as possibly other surgical procedures). In this regard, treatment of a tumor in accordance with aspects of the present disclosure includes forming an access opening in the patient's skull (e.g., a conventional craniotomy). Once a target site at which the brain tumor has been exposed, the system 10 is operated to remove at least some, preferably all, of the brain tumor, regardless of whether it is a soft or a fibrous tumor. In accordance with aspects of the present disclosure, embodiments of the cutting implement 14 are particularly suitable for removal of tumors at the interface of the tumor and viable tissue, although the embodiments are suitable for use elsewhere as well.

The aggressiveness of the tissue removal can be controlled by the size and blade configuration as well as the controlled amount of aspiration. The cutting windows 128, 228 are open to the aspiration pathway at all times. For example, as the surgeon approaches an area of the patient's brain that a tumor is to be removed, the surgical cutting instrument 10 is moved generally toward the target site with the surgeon's finger removed from the aspiration control hole 34; in this manner, there is no negative pressure, or aspiration at the target site. As the cutting implement 14 nears the target site and the surgeon desires to approximate the location of the cutter 26 to the target site, the surgeon can gradually or fully close down by partially or fully covering the aspiration control port 34 with their finger; in this manner, the negative pressure, or suction, provides intimate contact of the tissue with the cutter 26. This allows for neurosurgery or spine surgery on the dura without damage to the dura.

Once the surgeon positions the cutter 26 of the cutting implement 14 adjacent the tumor, the surgeon manipulates the handpiece 12 so as to position the cutting window 118, 228 adjacent and/or into the brain tumor. Rotation of the inner blade 22', 22" of the cutting implement 14 is effectuated by activation through the IPC (not shown). The controller/IPC (via a connection between the handpiece 12) enables selective rotational control over the inner blade 22', 22" to cause high-speed rotation of the cutting implement 14 for debriding or otherwise cutting the target tissue. The location of the cutting window 118, 228 at the distal end 124, 224 with openings in both the terminal end and side walls provides the ability to resect fibrous tissue in lateral planes as well as end planes. Depending upon the particular location of the tumor, the cutting implement 14 can be manipulated sideways across the tumor or downward without overtly twisting/contortion of the surgeon's hand(s).

Minimal deflection occurs, in one embodiment less than 0.008 inches, as the inner blade 22', 22" and the associated cutting tip 126, 226 are rotated in a single (i.e., nonoscillating) direction during the cutting procedure, providing stability to the cutting implement 14. The cutting tip 126, 226 rotates as the target tissue is drawn toward the cutter 26 with the negative pressure such that the tissue contacts the cutting tip 126, 226 and the tissue is cleanly and neatly cut (i.e., without tearing) against the edges of the cutting window 118, 228 to minimize collateral damage to the surrounding tissue. The cutting tip 126, 226 takes bites of tissue to effectively emulsify the targeted tissue. The fluid source has been activated prior to rotation of the inner blade 22', 22". The inner blade 22', 22" is sized to accommodate the emulsified tissue, and thus, due to the small size of the tissue being removed through the inner blade 22', 22", the inner blade 22', 22" can be smaller than that of other debridders. Fluid from the fluid source assists with the emulsification and aspiration of the tumor. By controlling (minimizing) the aspiration at the cutting implement 14, unnecessary damage to the surrounding tissue is avoided. Additionally, due to the onedirectional rotation of the cutting implement 14, the surgeon can precisely locate and maintain the cutting implement 14 at the desired tumor.

As discussed above, the irrigation path extends between the inner and outer blades 22', 24' and inner and outer blades 22", 24", respectively. The irrigation fluid then can exit the cutting window 128 or 228, respectively, to irrigate the target site. The irrigation fluid path provides constant irrigation of the target site and a liquid medium for material being cut and removed through the central lumen of the inner blade 22' or 22". The aspiration control hole 34 allows the surgeon to control the negative pressure at the cutting tip 126, 226. The cutting windows 128, 228 and the lumen of the inner member 22', 22" serve as an aspiration outlet of the aspiration fluid pathway (FIG. 1) otherwise employed for aspirating a target site. Aspiration is manually controlled with the aspiration control hole 34 on the handpiece 12.

In some embodiments, the cutting implement 14 can be disassembled for cleaning or other purposes. In particular, the inner blade 22, 22', 22" can be disassembled the outer blade 24, 24', 24", respectively.

Although the present disclosure has been described with reference to preferred embodiments, workers skilled in the art will recognize that changes can be made in form and detail without departing from the scope of the present invention as defined by the claims.

## Claims

1. A cutting device for use with a powered surgical tool, comprising:
an outer blade (24) including a tubular body having side walls, a planar distal end extending at an angle to the side walls, and a cutting window (128) formed in the distal end and the side walls; and
an inner blade (22) having a distal portion (36) and an intermediate portion (38) extending proximally from the distal portion, the distal portion including a cutting tip (126) having side and end cutting surfaces forming an opening (140), wherein the distal portion includes an outer surface having a first outer diameter and the intermediate portion includes an exterior surface having a second outer diameter, wherein the first outer diameter is greater than the second outer diameter,
wherein the inner blade is co-axially disposed within the outer blade with the cutting tip being rotatably exposed at the cutting window, and wherein the cutting tip directly contacts the distal end of the outer blade;
**characterized in that** the distal portion (36) includes a first irrigation channel (246) extending longitudinally from the opening to terminate at the intermediate portion, wherein the first irrigation channel is a planar surface that is recessed from the first outer diameter into a thickness of the distal portion.

2. The cutting device of claim 1, wherein an interior surface of the distal end intersects an inner surface of the side walls at a 90° angle.

3. The cutting device of claim 1 or 2, wherein the cutting window includes an outwardly beveled edge and the cutting tip includes an inwardly beveled edge.

4. The cutting device of any preceding claim, wherein the distal portion includes a second irrigation channel (246) formed within a thickness of the distal portion.

5. The cutting device of claim 4, wherein the second irrigation channel extends along the distal portion and is aligned with openings of the cutting tip.

6. The cutting device of any preceding claim, wherein a central lumen of the inner blade is continuously fluidly open at the cutting window.

7. The cutting device of any preceding claim, wherein the cutting tip includes teeth (136a, 136b) extending toward each other across an opening, and a U-shaped opening spaced from the teeth.

8. The cutting device of any preceding claim, further comprising a window orientation hub (58) secured to the outer blade and configured to adjust the position of the cutting window.

9. A cutting device for use with a powered surgical tool, comprising:
an outer blade (24) including a tubular body, a planar distal end cap, and a cutting window (128) formed in the distal end cap;
an inner blade (22) including a cutting tip (126) forming an opening (140), a distal portion (36) and an intermediate portion (38) at the distal portion, wherein the inner blade is co-axially disposed within the outer blade along a longitudinal axis with the cutting tip is rotatably exposed at the cutting window, wherein the distal portion includes an outer surface having a first outer diameter and the intermediate portion includes an exterior surface having a second outer diameter;
an outer hub assembly (50) assembled to the outer blade; and
an inner hub assembly (42) assembled to the inner blade, wherein the inner hub assembly biases the cutting tip toward the distal end cap;
**characterized in that**
wherein the first outer diameter is greater than the second outer diameter, wherein the distal portion includes a first irrigation channel (246) extending longitudinally from the opening to terminate at the intermediate portion, wherein the first irrigation channel is a planar surface that is recessed from the first outer diameter into a thickness of the distal portion

10. The cutting device of claim 9, further comprising a window orientation assembly (20) configured to selectively orient the cutting window radially around the longitudinal axis with respect to the inner hub assembly and the outer hub assembly.

11. The cutting device of claim 10, wherein the window orientation assembly comprises:
a tubular shaft (60) having a proximal end and a distal end, the tubular shaft coaxially extending around the outer blade, the proximal end disposed adjacent to a proximal section of outer blade and within the outer hub assembly;
a window orientation hub (58) disposed around the distal end of the tubular shaft; and
a sealing ring (110) disposed around the distal end of the outer blade to fluidly seal the outer blade and the tubular shaft.

12. The cutting device of claim 11, wherein window orientation hub is disposed along the outer blade adjacent to the outer hub assembly.

13. The cutting device of claim 11, wherein the window orientation hub is configured for manual manipulation.

14. The cutting device of claim 9, wherein an interior surface of the distal end cap intersects an inner surface of the tubular body at a 90° angle.

15. The cutting device of claim 9, wherein the inner hub assembly includes a spring, and wherein the spring biases the inner blade toward the distal end cap.

## Patentansprüche

1. Schneidevorrichtung zur Verwendung mit einem angetriebenen chirurgischen Werkzeug, umfassend:
eine äußere Klinge (24), die einen rohrförmigen Körper mit Seitenwänden, ein planares distales Ende, das sich in einem Winkel zu den Seitenwänden erstreckt und ein Schneidefenster (128), das in dem distalen Ende und den Seitenwänden ausgebildet ist, einschließt; und
eine innere Klinge (22), die einen distalen Abschnitt (36) und einen Zwischenabschnitt (38) aufweist, der sich proximal von dem distalen Abschnitt erstreckt, wobei der distale Abschnitt eine Schneidespitze (126) mit Seiten- und Endschneideoberflächen einschließt, die eine Öffnung (140) bilden, wobei der distale Abschnitt eine Außenoberfläche mit einem ersten Außendurchmesser einschließt und der Zwischenabschnitt eine Außenoberfläche mit einem zweiten Außendurchmesser einschließt, wobei der erste Außendurchmesser größer als der zweite Außendurchmesser ist,
wobei die innere Klinge koaxial innerhalb der äußeren Klinge angeordnet ist, wobei die Schneidespitze am Schneidefenster drehbar freiliegt, und wobei die Schneidespitze das distale Ende der äußeren Klinge direkt berührt;
**dadurch gekennzeichnet, dass** der distale Abschnitt (36) einen ersten Spülkanal (246) einschließt, der sich in Längsrichtung von der Öffnung erstreckt, um an dem Zwischenabschnitt zu enden, wobei der erste Spülkanal eine ebene Oberfläche ist, die von dem ersten Außendurchmesser in eine Dicke des distalen Abschnitts vertieft ist.

2. Schneidevorrichtung nach Anspruch 1, wobei eine Innenoberfläche des distalen Endes eine Innenoberfläche der Seitenwände in einem 90°-Winkel schneidet.

3. Schneidevorrichtung nach Anspruch 1 oder 2, wobei das Schneidefenster eine nach außen abgeschrägte Kante einschließt und die Schneidespitze eine nach innen abgeschrägte Kante einschließt.

4. Schneidevorrichtung nach einem der vorhergehenden Ansprüche, wobei der distale Abschnitt einen zweiten Spülkanal (246) einschließt, der innerhalb einer Dicke des distalen Abschnitts ausgebildet ist.

5. Schneidevorrichtung nach Anspruch 4, wobei sich der zweite Spülkanal entlang des distalen Abschnitts erstreckt und mit Öffnungen der Schneidespitze ausgerichtet ist.

6. Schneidevorrichtung nach einem der vorhergehenden Ansprüche, wobei ein zentrales Lumen der inneren Klinge am Schneidefenster kontinuierlich fluidisch offen ist.

7. Schneidevorrichtung nach einem der vorhergehenden Ansprüche, wobei die Schneidespitze Zähne (136a, 136b), die sich über eine Öffnung zueinander erstrecken, und eine U-förmige Öffnung, die von den Zähnen beabstandet ist, einschließt.

8. Schneidevorrichtung nach einem der vorhergehenden Ansprüche, ferner umfassend eine Fensterausrichtungsnabe (58), die an der äußeren Klinge befestigt und konfiguriert ist, um die Position des Schneidefensters einzustellen.

9. Schneidevorrichtung zur Verwendung mit einem angetriebenen chirurgischen Werkzeug, umfassend:
eine äußere Klinge (24), die einen rohrförmigen Körper, eine planare distale Endkappe und ein Schneidefenster (128) einschließt, das in der distalen Endkappe ausgebildet ist;
eine innere Klinge (22), die eine Schneidespitze (126) einschließt, die eine Öffnung (140), einen distalen Abschnitt (36) und einen Zwischenabschnitt (38) an dem distalen Abschnitt bildet, wobei die innere Klinge koaxial innerhalb der äußeren Klinge entlang einer Längsachse angeordnet ist, wobei die Schneidespitze drehbar am Schneidefenster freiliegt, wobei der distale Abschnitt eine Außenoberfläche mit einem ersten Außendurchmesser einschließt und der Zwischenabschnitt eine Außenoberfläche mit einem zweiten Außendurchmesser einschließt;
eine äußere Nabenanordnung (50), die an der äußeren Klinge montiert ist; und
eine innere Nabenanordnung (42), die an der inneren Klinge montiert ist, wobei die innere Nabenanordnung die Schneidespitze in Richtung der distalen Endkappe vorspannt;
**dadurch gekennzeichnet, dass**
wobei der erste Außendurchmesser größer als der zweite Außendurchmesser ist, wobei der distale Abschnitt einen ersten Spülkanal (246) einschließt, der sich in Längsrichtung von der Öffnung erstreckt, um an dem Zwischenabschnitt zu enden, wobei der erste Spülkanal eine planare Oberfläche ist, die von dem ersten Außendurchmesser in eine Dicke des distalen Abschnitts vertieft ist.

10. Schneidevorrichtung nach Anspruch 9, ferner umfassend eine Fensterausrichtungsanordnung (20), die konfiguriert ist, um das Schneidefenster selektiv radial um die Längsachse in Bezug auf die innere Nabenanordnung und die äußere Nabenanordnung auszurichten.

11. Schneidevorrichtung nach Anspruch 10, wobei die Fensterausrichtungsanordnung umfasst:
Eine rohrförmige Welle (60) mit einem proximalen Ende und einem distalen Ende, wobei sich die rohrförmige Welle koaxial um die äußere Klinge erstreckt, wobei das proximale Ende angrenzend an einen proximalen Abschnitt der äußeren Klinge und innerhalb der äußeren Nabenanordnung angeordnet ist;
eine Fensterausrichtungsnabe (58), die um das distale Ende des rohrförmigen Schafts herum angeordnet ist; und
einen Dichtungsring (110), der um das distale Ende der äußeren Klinge herum angeordnet ist, um die äußere Klinge und die rohrförmige Welle fluidisch abzudichten.

12. Schneidevorrichtung nach Anspruch 11, wobei die Fensterausrichtungsnabe entlang der äußeren Klinge angrenzend an die äußere Nabenanordnung angeordnet ist.

13. Schneidevorrichtung nach Anspruch 11, wobei die Fensterausrichtungsnabe für manuelle Manipulation konfiguriert ist.

14. Schneidevorrichtung nach Anspruch 9, wobei eine Innenoberfläche der distalen Endkappe eine Innenoberfläche des rohrförmigen Körpers in einem 90°-Winkel schneidet.

15. Schneidevorrichtung nach Anspruch 9, wobei die innere Nabenanordnung eine Feder einschließt, und wobei die Feder die innere Klinge in Richtung der distalen Endkappe vorspannt.

## Revendications

1. Dispositif de coupe destiné à être utilisé avec un outil chirurgical motorisé, comprenant :
une lame extérieure (24) comportant un corps tubulaire ayant des parois latérales, une extrémité distale plane s'étendant à un angle par rapport aux parois latérales, et une fenêtre de coupe (128) formée dans l'extrémité distale et les parois latérales ; et
une lame intérieure (22) ayant une partie distale (36) et une partie intermédiaire (38) s'étendant de manière proximale à partir de la partie distale, la partie distale comportant une pointe de coupe (126) ayant des surfaces de coupe latérales et d'extrémité formant une ouverture (140), la partie distale comportant une surface extérieure ayant un premier diamètre extérieur et la partie intermédiaire comportant une surface extérieure ayant un second diamètre extérieur, le premier diamètre extérieur étant supérieur au second diamètre extérieur,
dans lequel la lame intérieure est disposée de manière coaxiale à l'intérieur de la lame extérieure, la pointe de coupe étant exposée de manière rotative au niveau de la fenêtre de coupe, et la pointe de coupe étant directement en contact avec l'extrémité distale de la lame extérieure ;
**caractérisé en ce que** la partie distale (36) comporte un premier canal d'irrigation (246) s'étendant longitudinalement depuis l'ouverture pour s'arrêter au niveau de la partie intermédiaire, dans lequel le premier canal d'irrigation est une surface plane qui est en retrait à partir du premier diamètre externe en une épaisseur de la partie distale.

2. Dispositif de coupe selon la revendication 1, dans lequel une surface intérieure de l'extrémité distale coupe une surface intérieure des parois latérales à un angle de 90°.

3. Dispositif de coupe selon la revendication 1 ou 2, dans lequel la fenêtre de coupe comporte un bord biseauté vers l'extérieur et la pointe de coupe comporte un bord biseauté vers l'intérieur.

4. Dispositif de coupe selon l'une quelconque revendication précédente, dans lequel la partie distale comporte un second canal d'irrigation (246) formé dans une épaisseur de la partie distale.

5. Dispositif de coupe selon la revendication 4, dans lequel le second canal d'irrigation s'étend le long de la partie distale et est aligné avec des ouvertures de la pointe de coupe.

6. Dispositif de coupe selon l'une quelconque revendication précédente, dans lequel une lumière centrale de la lame intérieure est ouverte de manière continue au niveau de la fenêtre de coupe.

7. Dispositif de coupe selon l'une quelconque revendication précédente, dans lequel la pointe de coupe comporte des dents (136a, 136b) s'étendant l'une vers l'autre à travers une ouverture, et une ouverture en forme de U éloignée des dents.

8. Dispositif de coupe selon l'une quelconque revendication précédente, comprenant en outre un moyeu d'orientation de fenêtre (58) fixé à la lame extérieure et conçu pour ajuster la position de la fenêtre de coupe.

9. Dispositif de coupe destiné à être utilisé avec un outil chirurgical motorisé, comprenant :
une lame extérieure (24) comportant un corps tubulaire, un capuchon d'extrémité distale plane et une fenêtre de coupe (128) formée dans le capuchon d'extrémité distale ;
une lame intérieure (22) comportant une pointe de coupe (126) formant une ouverture (140), une partie distale (36) et une partie intermédiaire (38) au niveau de la partie distale, la lame intérieure étant disposée de manière coaxiale à l'intérieur de la lame extérieure le long d'un axe longitudinal avec la pointe de coupe qui est exposée de manière rotative au niveau de la fenêtre de coupe, la partie distale comportant une surface extérieure ayant un premier diamètre extérieur et la partie intermédiaire comportant une surface extérieure ayant un second diamètre extérieur ;
un ensemble de moyeu externe (50) assemblé à la lame extérieure ; et
un ensemble de moyeu interne (42) assemblé à la lame intérieure, dans lequel l'ensemble de moyeu interne sollicite la pointe de coupe vers le capuchon d'extrémité distale ;
**caractérisé en ce que**
dans lequel le premier diamètre externe est supérieur au second diamètre externe, dans lequel la partie distale comporte un premier canal d'irrigation (246) s'étendant longitudinalement depuis l'ouverture pour s'arrêter au niveau de la partie intermédiaire, dans lequel le premier canal d'irrigation est une surface plane qui est en retrait à partir du premier diamètre externe dans une épaisseur de la partie distale.

10. Dispositif de coupe selon la revendication 9, comprenant en outre un ensemble d'orientation de fenêtre (20) conçu pour orienter sélectivement la fenêtre de coupe radialement autour de l'axe longitudinal par rapport à l'ensemble de moyeu interne et à l'ensemble de moyeu externe.

11. Dispositif de coupe selon la revendication 10, dans lequel l'ensemble d'orientation de fenêtre comprend :
un arbre tubulaire (60) ayant une extrémité proximale et une extrémité distale, l'arbre tubulaire s'étendant de manière coaxiale autour de la lame extérieure, l'extrémité proximale étant disposée adjacente à une section proximale de la lame extérieure et à l'intérieur de l'ensemble de moyeu externe ;
un moyeu d'orientation de fenêtre (58) disposé autour de l'extrémité distale de l'arbre tubulaire ; et
une bague d'étanchéité (110) disposée autour de l'extrémité distale de la lame extérieure pour sceller fluidiquement la lame extérieure et l'arbre tubulaire.

12. Dispositif de coupe selon la revendication 11, dans lequel le moyeu d'orientation de fenêtre est disposé le long de la lame extérieure adjacente à l'ensemble de moyeu externe.

13. Dispositif de coupe selon la revendication 11, dans lequel le moyeu d'orientation de fenêtre est conçu pour une manipulation manuelle.

14. Dispositif de coupe selon la revendication 9, dans lequel une surface intérieure du capuchon d'extrémité distale coupe une surface intérieure du corps tubulaire à un angle de 90°.

15. Dispositif de coupe selon la revendication 9, dans lequel l'ensemble de moyeu interne comporte un ressort, et dans lequel le ressort sollicite la lame intérieure vers le capuchon d'extrémité distale.
